# EUROPEAN PATENT APPLICATION

(11) **EP 2 544 256 A1**
(43) Date of publication of application: **09.01.2013**
(21) Application number: 11172559.4
(22) Date of filing: 04.07.2011
(51) Int. Cl.: H01L 51/00, C01B 31/02

(54) **Two-component electron-selective buffer layer and photovoltaic cells using the same**

(71) Applicant: LANXESS Deutschland GmbH, 51369 Leverkusen (DE)
(72) Inventor: Levin, Roman E., 142432 Chernogolovka, Moscow (RU); Kornev, Alexey B., 142432 Chernogolovka, Moscow (RU); Troshin, Pavel A., 142432 Chernogolovka, Moscow (RU); Razumov, Vladimir F., 142432 Chernogolovka, Moscow (RU)

(57) **Abstract**

The present invention relates to the use of certain substituted fullerenes in optoelectronic devices, preferably in photovoltaic cells, preferably in organic photovoltaic cells especially preferred in a two-component electron-selective buffer layer of an organic photovoltaic cell to improve the efficiency of solar cells for energy generation.

## Description

The present invention relates to the use of certain substituted fullerenes in optoelectronic devices, preferably in photovoltaic cells, preferably in organic photovoltaic cells, especially preferred in a two-component electron-selective buffer layer of an organic photovoltaic cell to improve the efficiency of solar cells for energy generation.

Photovoltaic devices allow for the most simple and efficient conversion of the solar energy to electricity. The first generation of solar cells based on crystalline silicon is known since the middle of the last century. However, wide scale distribution of such devices has been long time limited by their extremely high cost. The typical installation cost of the solar cells based on crystalline silicon technologies stays in the range 2-3 USD per every watt of energy generated at maximal (peak) solar irradiance (denoted as watt-peak, Wₚ). Organic solar cells are expected be able to produce electricity at the cost of around 20 cents per Wₚ. This level might be approached by implementation of the devices yielding reasonably high power conversion efficiencies of 8-16% at very low module costs (40-60 USD/m²). Indeed, laboratory prototypes of organic solar cells demonstrated power conversion efficiencies exceeding 8% and approaching 11% in the case of dye-sensitized solar cells. Further improvements of organic solar cells in terms of performance, life-time, module design and production technologies might lead to a breakthrough in the renewable energies. At the end, the energy generated by solar light conversion should become cheaper than the energy that we currently produce by combustion of fossil fuels.

There are also many additional advantages of the organic thin film solar cells that can be illustrated as follows.
- Mechanical flexibility allows one to adapt them to any curved surfaces;
- Light weight nature of thin film solar cells makes them ideal suiting for portable electronics applications;
- Integration into cloths (power-suite) and military canopies has been already demonstrated;
- High sensitivity at low light intensities allows for indoor applications to collect scattered light (e.g. use them as decorative energy-generating wall-paper).

Different examples of organic solar cells have entered the phase of commercialization recently. However, the market potential of organic solar cells is limited by their relatively low power conversion efficiency and short life times. Therefore, substantial improvements of the photoactive material combinations and the device architectures are required.

One of the severest problems limiting the performance of organic solar cells is the charge recombination at the active layer/electrode interfaces. In particular, transparent indium-tin oxide electrode (ITO) is used in all reasonably efficient organic photovoltaic cells designed by now. Due to its electronic nature the ITO material can extract both positive and negative charges from the active layer of the device. At the same time, top electrodes composed of aluminum or silver can also extract both holes and electrons. Such poor selectivity of the charge collection results in a low photovoltaic performance of the device because of the massive charge recombination at the electrodes. To avoid this loss, some buffer layers should be introduced at the interfaces between the electrodes and the active layer. Electron blocking functions were revealed for vacuum processed vanadium (V), molybdenum (VI) and tungsten (VI) oxides. Titanium dioxide, cesium carbonate, zinc oxide or fullerene derivatives behave as electron transporting and hole blocking materials.

It is illustrated by a number of examples and also by embodiments of the present invention that metal oxides form metastable interfaces with organic materials. This is particularly the problem of such sensitive photoactive components as conjugated polymers (especially ones with low band gaps) that can be easily doped or oxidized by oxygen and/or high-valence metal oxides. Therefore, it is reasonable to design and apply some additional organic interlayers that can improve the interfaces between the metal oxide buffer layers and photoactive layer of the device.

The use of fullerenes in photovoltaic cells was already described in DE 19 515 305 A1. The use of substituted fullerenes in self-organizid buffer layers in organic solar cells is known from Adv. Mater. 2008, 20, 2211-2216.

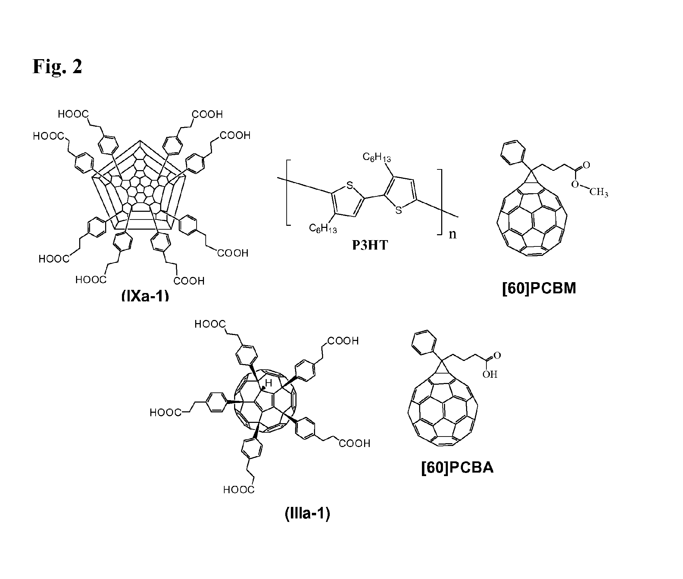

J.Am. Chem. Soc. 2010, 132, 4887-4893 describes the use of [6,6]-phenyl-C₆₁-butyric acid methylester (PCBM) (see Fig.2) to enhance the high power-conversion efficiencies in polymeric solar cells.

J.Am. Chem. Soc. 2010, 132, 17381-17383 exhibits the use of a poly(3-hexylthiophene) (P3HT)-based inverted solar cell using indene-[60] bis-adduct (ICBA) as the acceptor (see Fig.2).

Applied Materials & Interfaces, Vol. 2, No. 7, 1892-1902, 2010 discloses the manufacture of [60]-substituted benzoic acid (SAM[5]) and its use in ITO electrode based inverted solar cells.

As the use of fullerenes in the electron transporting (accepter-type) layer of organic-based photosensitive optoelectronic devices was already know from WO 02/101838 A1 even the problem of metal oxides forming metastable interfaces with organic materials was tried to be solved.

US 6 380 027 B2 discloses the use of fullerenes in solar cells. The use of Bis-[70]-PCBM and Bis-[60]-PCBM (see Fig.2) in photovoltaic cells is described in US 2010/0224252 A1. The use of fullerenes in the active layer-N-type material of photovoltaic cells is described in US 2010/0043876 A1. Solution processed squarine/[60] bilayer photovoltaic cells are disclosed in US 2010/0056112 A1 using two-component buffer layers consisting of n-type metal oxide covered with thin layers of cross-linkable fullerene derivatives according to formulae 1 or 2. This approach has few disadvantages. First, cross-linking of the fullerene derivatives of formulae 1 and 2 is a slow procedure which is hardly compatible with industrial processes. Second, radical or cationic cross-linking results in the formation of numerous defect sites in the material that typically serve as traps for charge carriers.

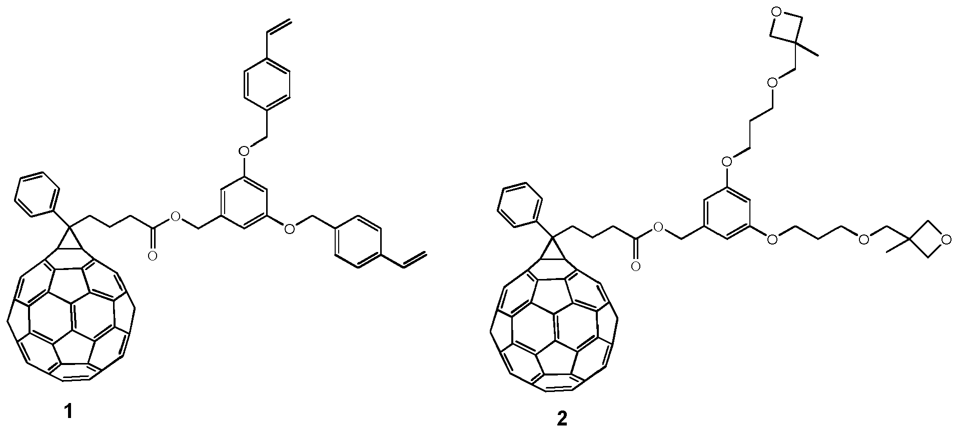

Alternative two-component electron selective buffer layers were based on n-type metal oxides covered with self-assembled monolayers of the fullerene derivatives according to formulae **3** to **6** (J. Mater. Chem., 2008, 18, 5113-5119; Appl. Phys. Lett., 2008, 93, 233304; ACS Appl. Mater. Interfaces, 2010, 2, 1892). All these compounds of formulae **3** to **6** possess a carboxylic or phosphonic acid group which is capable of anchoring to the ZnO or TiO₂ surface. The disadvantage of the fullerene derivatives according to formulae **3** to **6** is the presence of just one anchoring carboxylic or phosphonic acid group in their molecular structure. Therefore, large fullerene moiety remains quite mobile on the fullerene surface and could be even partially washed away by some solvents thus disrupting the continuity of the monolayer coverage. Such processes create defects in the two-component buffer layer affecting the performance of photovoltaic devices.

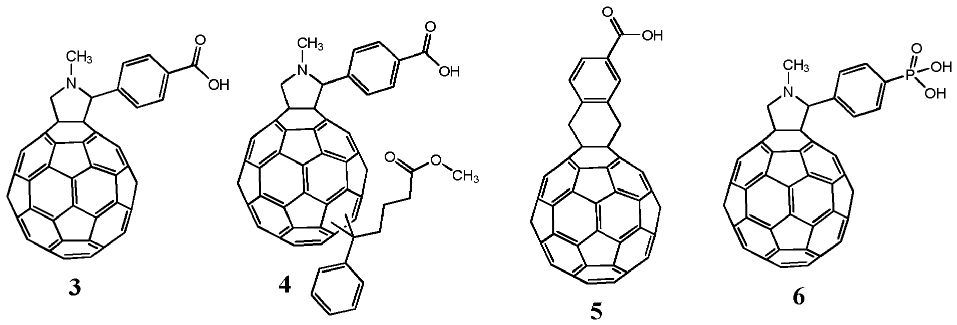

A [60] fullerene derivative of the formula 7 or its potassium salt and its use as an antiviral compound was published in Org. Biomol. Chem. 2007, 5, 2783 - 2791 by O.A. Troshina et. al.

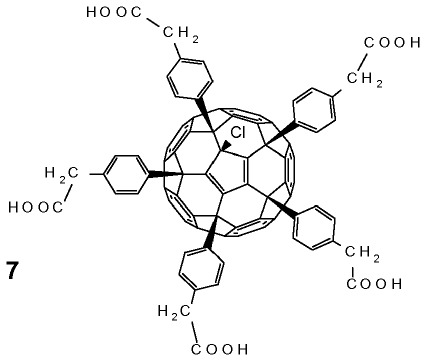

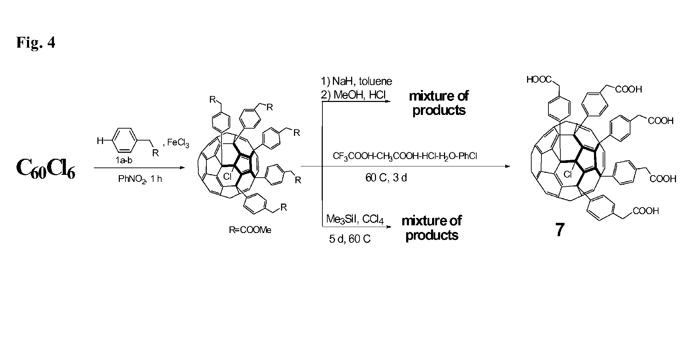

Its manufacture according to this reference is shown in Fig. 4.

With respect to the above described disadvantages of fullerene derivatives in organic solar cells it therefore was an object of the present invention to provide fullerene derivatives for the two-component electron-selective buffer layer in optoelectronic devices that do not show the mobility in the organic material with respect to solvents and that do not form defects in the two-component buffer layer while being industrially applicable in regard to their cross-linking behaviour.

The object is achieved by the use of compounds of the formula (I)

X-F-(Z-Y-M)ᵣ (I)

wherein
- F: is a [60] fullerene or [70] fullerene,
- M: represents COOH or P(O)(OH)₂,
- r: represents a number from 2 to 8,
- Z: represents a group -(CH₂)ₙ-, Ar, -CR(R')- or -S-,
- n: represents a number from 1 to 12,
- R and R': independent from each other represent hydrogen or a C₁-C₁₂ carbon chain,
- Y: represents a C₁-C₁₂ carbon chain,
- Ar: represents phenyl, biphenyl or naphthyl and
- X: represents H, Cl or independent from Y a C₁-C₁₂ carbon chain

in optoelectronic devices, preferably in organic photovoltaic cells, especially preferred in a two-component electron-selective buffer layer of such device.

For clarification, it should be noted that the scope of the invention encompasses all of the definitions and parameters listed in general terms or in preferred ranges in the present specification, in any desired combination. Additionally "[60] " represents a C60 fullerene and "[70]" represents a C70 fullerene.

In a preferred embodiment of the present invention when X represents a carbon chain this is a C₁-C₆ carbon chain.

In a preferred embodiment of the present invention Ar represents phenyl.

In a preferred embodiment of the present invention n represents a number from 1 to 6.

In a preferred embodiment of the present invention r = 5 in case of a [60] fullerene.

In a preferred embodiment of the present invention r = 8 in case of a [70] fullerene.

In a preferred embodiment of the present invention Y, R and R' each represent independent from another a C₁-C₆ carbon chain.

In a preferred embodiment the object is achieved by the use of [60] fullerenes of formula (II) 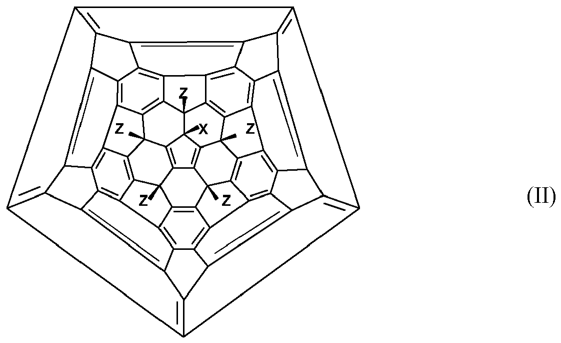 wherein
- X: represents H, Cl or independent from Y a C₁-C₁₂ carbon chain when
- Z: is a substituent selected from the group of -Ar-Y-COOH, -S-Y-COOH, -CR(R')-Y-COOH, -Ar-Y-P(O)(OH)₂, -S-Y-PO(OH)₂ or -CR(R')-Y-PO(OH)₂,
- Ar: represents phenyl, biphenyl or naphthyl,
- Y: is a C₁-C₁₂ carbon chain and
- R, R': each represents independent from each other hydrogen or a C₁-C₁₂ carbon chain
in optoelectronic devices, preferably in organic photovoltaic cells, especially preferred in a two-component electron-selective buffer layer of such device.

In a very preferred embodiment of the present invention all Z in a [60] fullerene represent the same substituent.

In a very preferred embodiment of the present invention Y in a [60] fullerene represents a)C₁-C₆ carbon chain.

In a very preferred embodiment of the present invention Ar in a [60] fullerene represents phenyl.

In a preferred embodiment the object is achieved by the use of [70] fullerenes of formula (IIa) 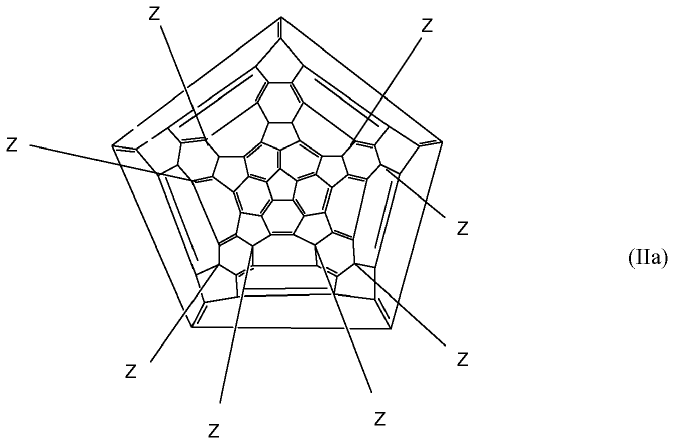 wherein
- Z: is a substituent selected from the group of -Ar-Y-COOH, -S-Y-COOH, -CR(R')-Y-COOH, -Ar-Y-P(O)(OH)₂, -S-Y-PO(OH)₂ or -CR(R')-Y-PO(OH)₂,
- Ar: represents phenyl, biphenyl or naphthyl,
- Y: is a C₁-C₁₂ carbon chain and
- R, R': each represents independent from each other hydrogen or a C₁-C₁₂ carbon chain
in optoelectronic devices, preferably in organic photovoltaic cells, especially preferred in a two-component electron-selective buffer layer of such device.

.In a very preferred embodiment of the present invention all Z in a [70] fullerene represent the same substituent.

In a very preferred embodiment of the present invention Y in a [70] fullerene represents a C₁-C₆ carbon chain.

In a very preferred embodiment of the present invention Ar in a [70] fullerene represents phenyl.

In a preferred embodiment of the present invention the fullerene derivative is represented by a fullerene-based compound 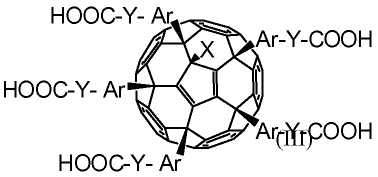 of general formula (III)
where Ar, X and Y have the above given meanings and Ar is directly bonded to the [60]fullerene carbon cage.

In a very preferred embodiment of the present invention the [60] fullerene derivative has formula (IIIa) 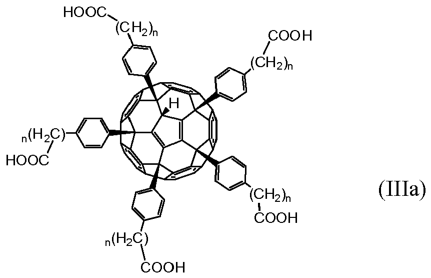 where n=1-12, preferably n = 1-6, especially preferred n = 2-4.

In a preferred embodiment of the present invention the fullerene derivative is represented by a [60] fullerene-based compound of general formula (IV) 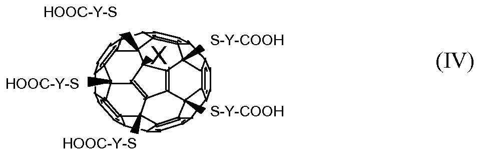 wherein
X and Y have the above given meanings.

In a very preferred embodiment of the present invention the [60] fullerene derivative has formula (IVa) 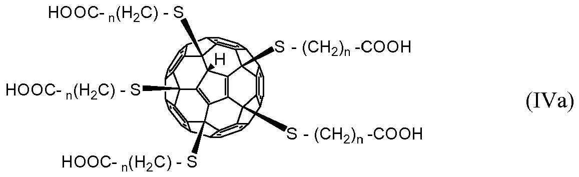 where n=1-12 and preferably n=1-2.

In a preferred embodiment of the present invention the fullerene derivative is represented by a [60] fullerene-based compound of general formula (V) wherein X, Y, R and R' have the above given meanings

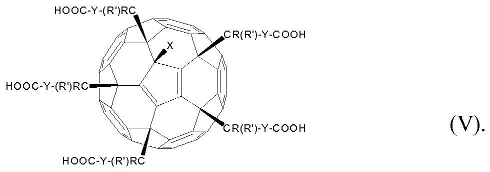

In a very preferred embodiment of the present invention the [60] fullerene derivative has formula (Va) 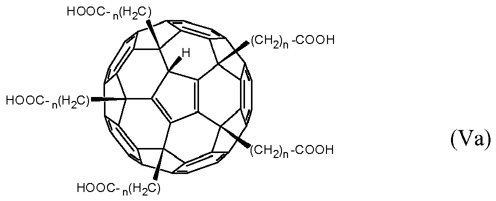 where n=1-12 and preferably n=1-3.

In a preferred embodiment of the present invention the fullerene derivative is represented by a [60] fullerene-based compound of general formula (VI) 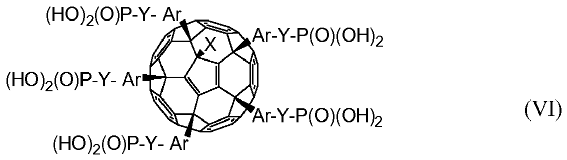 wherein X, Y and Ar have the above given meanings.

In a preferred embodiment of the present invention the fullerene derivative is represented by a [60] fullerene-based compound of general formula (VII) 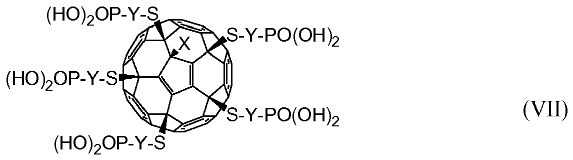 wherein X and Y have the above given meanings.

In a preferred embodiment of the present invention the fullerene derivative is represented by a [60] fullerene-based compound of general formula (VIII) 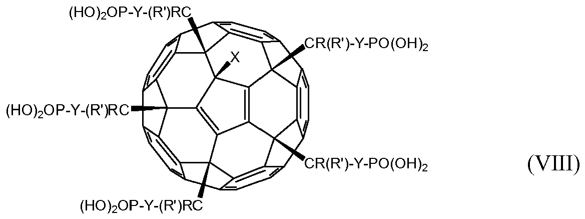 wherein X, Y, R and R' have the above given meanings.

In a preferred embodiment of the present invention the fullerene derivative is represented by a [70] fullerene-based compound of general formula (IX) 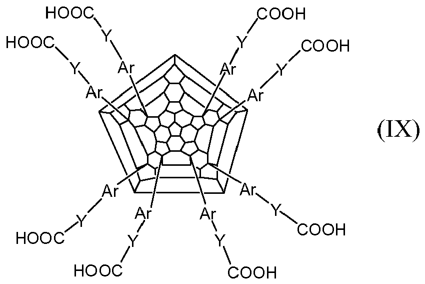 wherein Ar and Y have the above given meanings.

In a very preferred embodiment of the present invention the fullerene derivative is represented by a [70] fullerene-based compound of general formula (IXa) 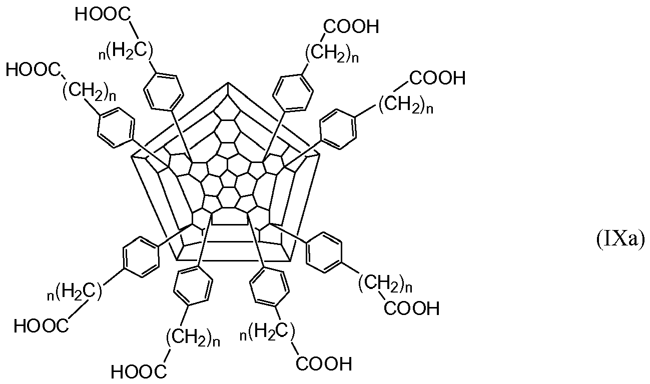 where n = 1-12 and preferably n = 1-3.

In a preferred embodiment of the present invention the fullerene derivative is represented by a [70] fullerene-based compound of general formula (X) where Ar and Y have the above given meanings.

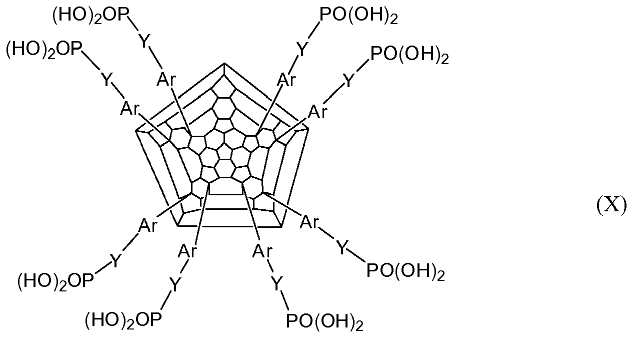

In a preferred embodiment of the present invention the above given fullerene derivatives are used in a mixture of at least any two compounds of general formulae (III) to (X) taken in any appropriate ratio.

In a very preferred embodiment of the present invention the above given fullerene derivatives are used in a mixture of at least any two compounds of general formulae (III) to (X) taken in any appropriate ratio in combination with 0.0001 to 99.9999% of a third component which might be represented by some functionalized higher fullerene C>70, some solvent, some processing additive or any other functional component improving or not affecting the performance of the fullerene derivatives in the claimed optoelectronic devices, preferably in organic photovoltaic cells, especially preferred in a two-component electron-selective buffer layer of such device.

Especially preferred the present invention refers to the use of fullerene derivatives (IIIa-1) and (IXa-1) in optoelectronic devices, preferably in a organic photo cell, especially preferred in a two-component electron-selective buffer layer.

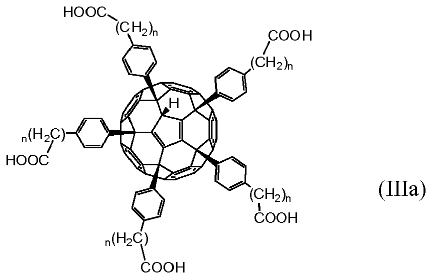

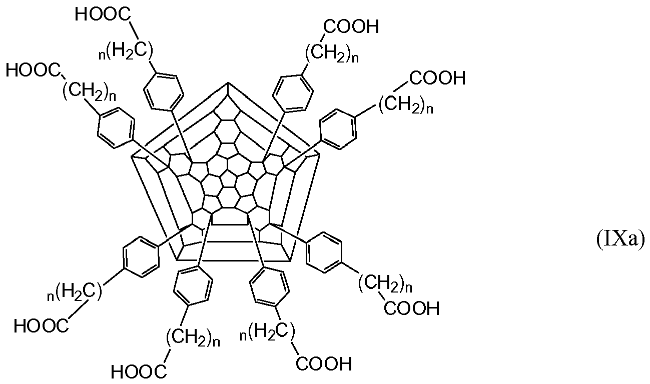

An object of the present inventions is also the use of the [60] fullerenes of the formulae (III) to (VIII) and the [70] fullerenes of the formulae (IX) and (X) in optoelectronic devices.

A preferred object of the invention is the use of those [60] fullerenes of the formulae (IIIa), (IVa), (Va) and the [70] fullerenes according to formula (IXa) in optoelectronic devices.

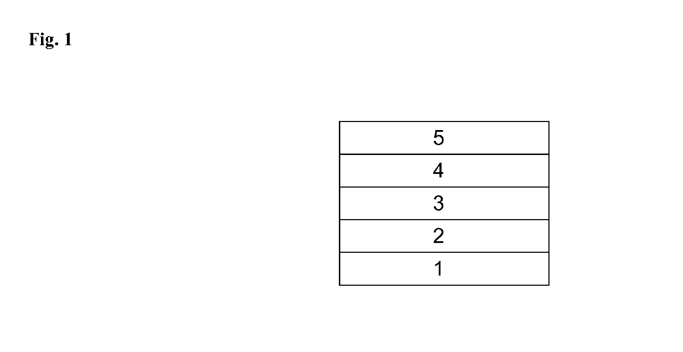

Preferred embodiments of the present invention are related to optoelectronic devices, preferably to organic photovoltaic cells that comprise at least one two-component electron selective buffer layer in their molecular architecture preferably, as shown in Fig. 1.

Fig. 1 is a schematic layout of a photovoltaic cell structure wherein the bottom electrode (1) is the collector of electrons from the device, the two-component electron selective buffer layer (2) is blocking the holes and non-dissociated excitations and conduction of electrodes towards the electrode (1); the active layer of photovoltaic cells functions as generator of free charge carriers under light irradiation; the hole selective layer is blocking electrons and non-dissociated excitations and conduction of holes towards the hole-collecting electrode (5).

This two-component electron selecting buffer layer functions as electron extracting and electron transporting layer in the device. In a preferred embodiment of the present invention the two-component electron selecting buffer layer consists of titanium dioxide (TiO₂) used as n-type semiconducting material and polycarboxylic derivative of [70] fullerene (IX-1) forming a self-assembled monolayer coverage on the TiO₂ surface exposed to the photoactive layer of the device. The implementation of this two-component electron selecting buffer layer structure improves fill factor (FF), short circuit current (I_{SC}), open circuit voltage (V_{OC}) and overall light power conversion efficiency (PCE) of organic photovoltaic cell in inverted configuration.

The term fill factor, as used in the present invention, refers to the ratio of the maximal electrical power produced by the device (Vₘₚ x Iₘₚ) divided by the short circuit current density (I_{SC}) and open circuit voltage (V_{OC}) in light-on current density - voltage characteristics of solar cells. The term short circuit current density (I_{SC}), as used herein, corresponds to the maximal current measured through the load under short-circuit conditions. The term open circuit voltage (V_{OC}), as used herein, is the maximal voltage obtainable at the load under open-circuit conditions. The term power conversion efficiency (PCE), as used herein, is the ratio of electrical power output from a device to the light power input (Pᵢₙ) defined as PCE = V_{OC} x I_{SC} x FF. The term inverted device configuration, as used herein, is the device structure where the transparent electrode (ITO, FTO, ATO or other) functions as an electron-collecting (negative) electrode. The terms classical or standard device configuration, as used herein, correspond to the device structure where the transparent electrode (ITO, FTO, ATO or other) functions as hole-collecting (positive) electrode.

An exemplary photovoltaic cell, according to an embodiment of the present invention, is the one where indium-tin oxide is used as bottom electrode (1), a two component buffer layer comprising TiO₂ and the fullerene derivative (IX-1) is serving as electron-selective buffer layer (2); poly(3-hexylthiophene)/[60]PCBM bulk heterojunction composite is used as the device active layer (3); MoO₃ applied as hole-selective buffer layer (4) and silver is used as counter electrode (5). The molecular structures of the materials are shown in Fig. 2.

In a preferred embodiment of the present invention other materials for the bottom electrode (1), electron-selective buffer layer (2), active layer (3), hole-selective layer (4) and top electrode (5) are used in addition to the ones presented in the disclosed example. In a preferred embodiment of the present invention, the active layer comprises any composite (blend or layer-by-layer structure) of electron-donating organic material and electron accepting organic material regardless their molecular weights and chemical compositions.

Preferred electron donor materials include conjugated polymers selected from the group poly(3-hexylthiophene) P3HT, poly(2,7-(9,9-di(alkyl)-fluorene)-alt-5,5-(4',7'-di-2-thienyl-2', 1',3'-benzothiadiazole)) (PFDTBT), poly(2,6-(4,4-bis-(2'-ethylhexyl)-4H-cyclopenta(2,1-b;3,4-6')dithiophene)-alt-4',7'-(2',1',3'-benzothiadiazole) (PCPDTBT), poly(2,6-(4,4-di(n-dodecyl)-4H-cyclopenta(2,1-b;3,4,-6')dithiophene)alt-5,5-(4',7'-di-2-thienyl-2',1',3'-benzothiadiazole)), poly[N-9'-heptadecanyl-2,7-carbazole-alt-5,5-(4',7'-di-2-thienyl-2',1',3'-benzothiadiazole) (PCDTBT), low-molecular weight donor materials preferably zinc or copper phthalocyanines, thiophene oligomers, organic dyes and other organic compounds characterized by their ability to form stable cationic species under chemical, photo- or electrochemical oxidation. A range of donor materials can be extended also to inorganic nanoparticles preferably PbS, PbSe, PdTe and other colloidal nanocrystals capable of the electron donation to the appropriate acceptor compound under light irradiation.

The acceptor material can be represented by any functionalized fullerene derivatives. The fullerene can be [60], [70], higher fullerene C>70 or any mixture of [60] with [70], [70] with higher fullerenes or [60], [70] and higher fullerenes. The acceptor material can be also represented by conjugated polymers, particularly ones comprising naphthalene bisimide or perylene bisimide units capable of n-type transport. At the same time, any low or high molecular weight organic compound can serve as acceptor material if it gives stable anions under chemical, photo- or electrochemical reduction conditions. The range of available acceptor materials can be extended to inorganic nanoparticles preferably colloidal ZnO or TiO₂ or nanocrystals composed of inorganic n-type semiconductors, preferably CdS or CdSe or optionally others as cited in the prior art.

The two-component electron selective buffer layer as claimed in the present invention comprises metallic or n-type semiconductor metal oxide combined with a fullerene derivate of the above given formulae. Preferably n-type metal oxides include ZnO, TiO₂, SnO₂ and optionally some others, preferably ITO = indium-tin oxide, FTO = fluorine-doped tin-oxide and ATO = antimony-tin-oxide as well as some others. The range of applicable fullerene derivatives includes any of the above given fullerene-based compounds, preferably having 2-20 carboxylic or phosphonic acid groups attached via some organic linker to the carbon [60] or [70] cage, particularly preferred the compounds with general formulae (III) to (X) shown above.

A wide range of different materials might be used as hole-selective layers in photovoltaic devices. This include metal oxides in high valence state preferably WO₃, MoO₃, V₂O₅, NiO, Ag₂O. At the same time, the top electrode can be composed of any metal or transparent conductive metal oxide typically applied as conductive materials in the background prior art.

As described above, the present invention even preferably refers to an optoelectronic device, preferably a photovoltaic cell, having at least one two-component electron selective buffer layer in their molecular architecture according to Fig. 1 with a compound of formula (I).

In a preferred embodiment the electron-selective electrode (1) is transparent and placed adjacent to a transparent substrate (not shown in Fig. 1).

In a preferred embodiment of the present invention the hole-selective electrode (5) is transparent and placed adjacent to a transparent substrate (not shown in Fig. 1).

In another preferred embodiment of the present invention both the electron-selective electrode (1) and the hole-selective electrode (5) are transparent and one of them is placed adjacent to a transparent substrate (not shown in Fig. 1).

In another preferred embodiment of the present invention the two-component electron-selective buffer layer is composed of fullerene derivates according to formulae (I) or (II) and an inorganic oxide which displays n-type semiconductor properties.

In another preferred embodiment of the present invention the two-component electron-selective buffer layer is composed of a fullerene derivative according to formulae (I) or (II) and an inorganic oxide which displays metallic conductor properties.

In a very preferred embodiment of the present invention the n-type semiconductor inorganic oxide is represented by TiO₂, SnO₂ or ZnO.

In a very preferred embodiment of the present invention the metallic conductor oxide is represented by fluorine-doped (FTO), indium-doped (ITO) or antimony-doped (ATO) tin oxide.

In an especially preferred embodiment of the present invention the fullerene derivative is represented by a fullerene-based compound having 2-20 carboxylic groups attached via some organic linker to the carbon [60] or [70] cage.

In an especially preferred embodiment of the present invention the fullerene derivative is represented by a fullerene-based compound having 2-20 phosphonic acid groups attached via some organic linker to the carbon [60] or [70] cage.

In a very especially preferred embodiment of the present invention the fullerene derivate forms a self-assembled monolayer on the surface of the inorganic oxide.

Compounds of formula (III) and (IIIa) can be manufactured according to a process described in Org. Biomol. Chem., 2007, 5, 2783 - 2791. Compounds of formula (IV) and (IVa) can be manufactured according to a process described in Proceedings of the XXI Mendeleyev Competition of Students, 2004, Vol.1, page 55 in Russian language. Compounds of formula (V) and (Va) can be manufactured according to a process described in org. Lett. 2008, 10(4), 621 - 623.Compounds of formula (VI) can be manufactured according to a process described in Org. Biomol. Chem., 2007, 5, 2783 - 2791. Compounds of formula (VII) can be manufactured according to a process described in Proceedings of the XXI Mendeleyev Competition of Students, 2004, Vol. 1, page 55 in Russian language. Compounds of formula (VIII) can be manufactured according to a process described in Org. Lett. 2008, 10(4), 621 - 623. Compounds of formula (IX) and (IXa) can be manufactured according to a process described in Chemical Communications 2011, DOI: 10.1039/C1CC12209F. Compounds of formula (X) can be manufactured according to a process described in Chemical Communications 2011, DOI: 10.1039/C1CC12209F.

In case of a [60] fullerene the manufacture starts with the production of chlorofullerene [60] Cl₆ which was reported in 1993 being among the first halides discovered for [60] fullerene (J. Chem. Soc. Chem. Commun.; 1993, 1230). Another synthesis, the socalled "seven minute synthesis of pure [60] C1₆" is described in Chem. Eur. J., 2005, 11, 5326. Still another synthesis based on the use of KJCl₄ is described in Full. Nanot. Carb. Nanostruct. 2003, 11, 165.

Notable also is the application of POCl₃ for [60] fullerene chlorination according to Mendeleev Commun., 2006, 209-2010.

In a second step [60]Cl₆ is reacted with methylesters of phenylacetic acid, preferably in the presence of nitrobenzene, to give the methyl ester of the compounds of formula (III). For cleavage of the methyl group reference is given to J. Chem. So. Chem. Commun. 1994, 1727 or in J. Org. Chem., 1995, 60, 532.

Fig. 4 shows the reaction to obtain products of formula (III).

The present invention is further directed to the compounds of the formula (I)

X-F-(Z-Y-M)ᵣ (I)

wherein
- F: is a [60] fullerene,
- M: represents P(O)(OH)₂,
- r: represents a number from 2 to 8,
- Z: represents a group Ar, -CR(R')- or -S-,
- n: represents a number from 1 to 12,
- R and R': independent from each other represent hydrogen or a C₁-C₁₂ carbon chain,
- Y: represents a C₁-C₁₂ carbon chain,
- Ar: represents phenyl, biphenyl or naphthyl and
- X: represents H, Cl or independent from Y a C₁-C₁₂ carbon chain.

In a preferred embodiment the present invention is directed to the compounds of formula (VI).

In a preferred embodiment the present invention is directed to the compounds of formula (VII).

In a preferred embodiment the present invention is directed to the compounds of formula (VIII).

The present invention is further directed to the compounds of the formula (I)

X-F-(Z-Y-M)ᵣ (I)

wherein
- F: is a [70] fullerene,
- M: represents P(O)(OH)₂,
- r: represents a number from 2 to 8,
- Z: represents a group Ar,
- n: represents a number from 1 to 12,
- Y: represents a C₁-C₁₂ carbon chain,
- Ar: represents phenyl, biphenyl or naphthyl and
- X: represents H, Cl or independent from Y a C₁-C₁₂ carbon chain.

In a preferred embodiment the present invention is directed to the compounds of formula (X).

### Examples:

### Examples 1

A photovoltaic cell, according to the present invention, as illustrated in Fig. 1, can be constructed in the following way. The TiO₂ thin films were prepared starting from the tetrabutyl titanate Ti(OC₄H₉)₄ through a sol-gel method reported in App. Phys. Lett. 2008, 93, 193307. The procedure for the preparation of TiO₂-sol involved the dissolution of 10 ml of Ti(OC₄H₉)₄ in 60 ml ethanol C₂H₅OH followed by the addition of 5 ml of acetyl acetone. Then a solution composed of 30 ml of C₂H₅OH, 10 ml of de-ionized water, and 2 ml of hydrochloric acid (HCl) with the concentration of 0.28 mol/l was added dropwise under vigorous stirring. The resulting mixture was stirred at room temperature for additional 2 h. The patterned ITO-coated glass substrates were sonicated consecutively with acetone, isopropyl alcohol, and deionized water for 10 min. Subsequently TiO₂-sol was spin-coated on ITO-coated glass substrates at 3000 rpm. The resulting films were dried in air for 20 min and then were transferred to the chamber oven heated up to 450°C (means that the samples were brought into the hot oven). The annealing at 450°C takes typically 2 hrs. Annealed TiO₂ slides were sonicated additionally in distilled water (2-4 min) and isopropyl alcohol (5 min). The wet TiO₂-covered slides were transferred immediately into the solution of (IXa-1) in ethanol. The concentration of the (IXa-1) SAM modifier was kept on the level of ca. 0.1 mg/ml. The slides were kept overnight in the (IXa-1) solution. Afterwards, the slides with TiO₂ layer and deposited on the top (IXa-1) SAM were washed with pure isopropyl alcohol, dried and annealed inside the glove box at 120°C within 20 minutes. After annealing the films were washed one more time with isopropyl alcohol and dried with a stream of nitrogen. These slides were ready for the active layer deposition. The formula of (IXa-1) is given in Fig.2.

For the active layer deposition, a blend of 9 mg of PCBM and 12 mg of P3HT, both dissolved in 1 ml of chlorobenzene, was spin-coated at the spinning frequency of 900 rpm. The resulting films were annealed at 155°C for 3 min and then the devices were finalized by deposition of 5 nm of MoO₃ and 100 nm of Ag thus forming the hole-selective layer and the top electrode of the device. The device can be encapsulated using appropriate barrier foils and sealing adhesive materials.

To illustrate the improved performance of the photovoltaic cells according to the present invention, the current density - voltage (I-V) characteristics were examined for three sets of devices:
- solar cells comprising single-material electron selective buffer layer (TiO₂) (set **D1**);
- solar cells comprising two-component electron selective buffer layer composed of TiO₂ modified with self-assembled monolayer of fullerene derivative PCBA possessing only one carboxylic group in its molecular framework (set **D2**);
- solar cells comprising two-component electron selective buffer layers composed of TiO₂ modified with self-assembled monolayers of fullerene derivatives (IXa-1) and (IIIa-1) possessing multiple carboxylic groups in their molecular frameworks (set **D3**);

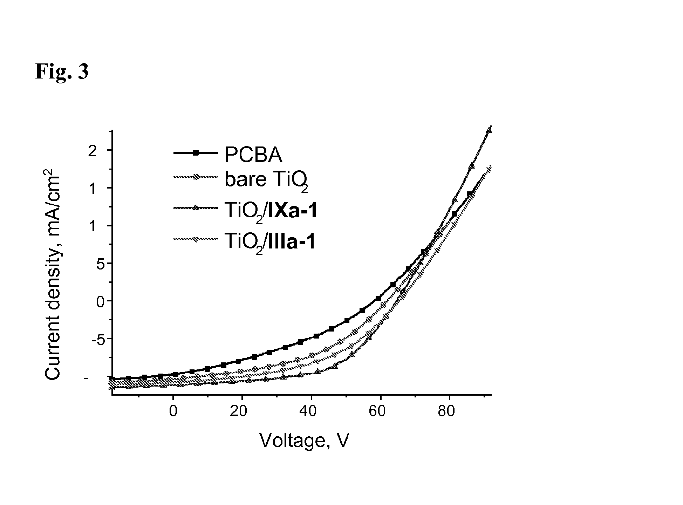

The obtained solar cell parameters are listed in Table 1. It is seen from the table that the device with a single-component electron selective buffer layer (TiO₂-only device) gives modest performance of about. 3.0 %. The application of fullerene derivative PCBA as monolayer modifier for TiO₂ does not improve the device performance. On the contrary, decrease of all device parameters was observed which suggests the formation of additional trap sites in the two-component electron selective layer. However, the application of fullerene derivatives (IIIa-1) and (IXa-1) results in significant enhancement of the device performance. In particular, the open circuit voltages and the fill factors go up which suggests the formation of very selective electron-collecting electrodes in these devices. Superior performance of the devices employing two-component electron-selective buffer layers comprising polycarboxylic fullerene derivatives (IIIa-1) and (IXa-1) is also well illustrated by the I-V curves presented in Fig. 3.

**Table 1 Photovoltaic characteristics of different types of photovoltaic devices**

| **Type** | **Fullerene derivative** | **Voc, mV** | **I_{SC}, mA/cm²** | **FF, %** | **PCE, %** |
|---|---|---|---|---|---|
| D1 | - | 618 | 10.3 | 47 | 3.0 |
| D2 | **PCBA** | 580 | 9.7 | 36 | 2.0 |
| D3 | **IIIa-1** | 654 | 10.7 | 50 | 3.5 |
| D3 | **IXa-1** | 650 | 11.2 | 58 | 4.2 |

Fig- 3. I-V shows curves of photovoltaic cells comprising different electron-selective buffer layers.

Considering the data presented in Table 1 one can notice that the devices comprising polycarboxylic fullerene derivatives (IIIa-1) and (IXa-1) give two times higher power conversion efficiencies (PCE) than the reference devices where PCBA possessing single carboxylic group was applied. At the same time, use of the two-component electron selective layers disclosed in the present invention improved the photovoltaic performance (FF) of the devices by 15-30% compared to the reference cells where bare TiO₂ was used as a buffer layer (=D1). Thus, the obtained results suggest that the two-component electron selective buffer layers composed of metal oxides and fullerene derivatives bearing 2-20 carboxylic or phosphonic acid groups as disclosed in the present invention might find broad range of applications in the field of organic photovoltaics. PCBA as used herein represents [60]PCBA according to Fig.2, CAS No. [161196-25-4], MW 896.85 available at IoLiTec Ionic Liquids Technologies GmbH, Heilbronn, Germany.

## Claims

1. Use of compounds of the formula (I)
X-F-(Z-Y-M)ᵣ (I)
wherein
F is a [60] fullerene or [70] fullerene,
M represents COOH or P(O)(OH)₂,
r represents a number from 2 to 8,
Z represents a group -(CH₂)ₙ-, Ar, -CR(R')- or -S-,
n represents a number from 1 to 12,
R and R' independent from each other represent hydrogen or a C₁-C₁₂ carbon chain,
Y represents a C₁-C₁₂ carbon chain,
Ar represents phenyl, biphenyl or naphthyl and
X represents H, Cl or independent from Y a C₁-C₁₂ carbon chain in optoelectronic devices.

2. Use according to Claim 1 wherein X represents a C₁-C₆ carbon chain.

3. Use according to Claims 1 or 2 wherein Ar represents phenyl.

4. Use according to Claims 1 to 3 wherein n represents a number from 1 to 6.

5. Use according to Claims 1 to 4 wherein r = 5 in case of a [60] fullerene.

6. Use according to Claims 1 to 4 wherein r = 8 in case of a [70] fullerene.

7. Use according to Claims 1 to 6 wherein Y, R and R' each represent independent from another C₁-C₆ carbon chain.

8. Use according to Claims 1 to 7 **characterized in that** a [60] fullerene of formula (II) 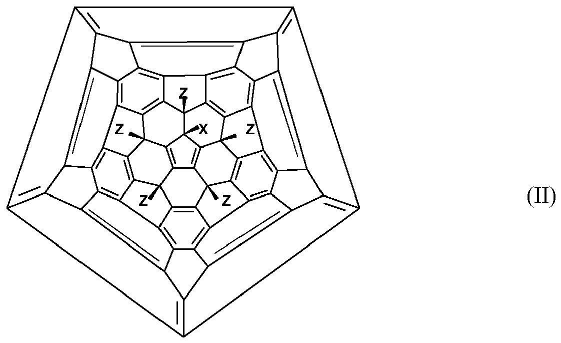 wherein
X represents H, Cl or independent from Y a C₁-C₁₂ carbon chain when
Z is a substituent selected from the group of -Ar-Y-COOH, -S-Y-COOH, -CR(R')-Y-COOH, -Ar-Y-P(O)(OH)₂, -S-Y-PO(OH)₂ or -CR(R')-Y-PO(OH)₂,
Ar represents phenyl, biphenyl or naphthyl,
Y is a C₁-C₁₂ carbon chain and
R, R' each represents independent from each other hydrogen or a C₁-C₁₂ carbon chain.

9. Use according to Claims 1 to 7 **characterized in that** a [70] fullerene of formula (IIa) 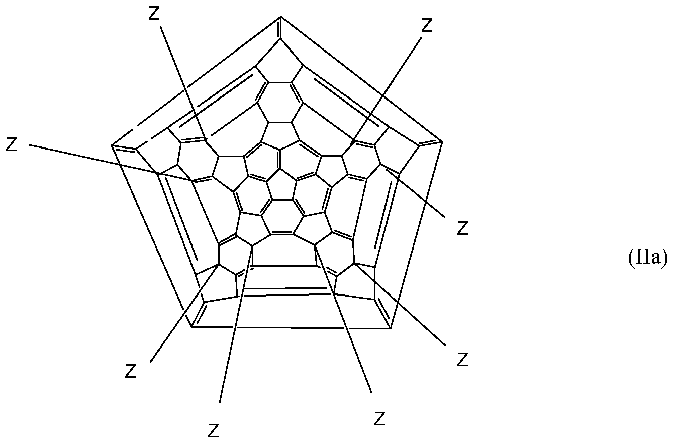 wherein
Z is a substituent selected from the group of -Ar-Y-COOH, -S-Y-COOH, -CR(R')-Y-COOH, -Ar-Y-P(O)(OH)₂, -S-Y-PO(OH)₂ or -CR(R')-Y-PO(OH)₂,
Ar represents an element from the group phenyl, biphenyl or naphthyl,
Y is a C₁-C₁₂ carbon chain and
R, R' each represents independent from each other hydrogen or a C₁-C₁₂ carbon chain is used in the two-component electron-selective butter layer of an optoelectronic device.

10. Use according to Claims 8 or 9 **characterized in that** all Z represent the same group.

11. Use according to Claims 1 to 10 **characterized in that** the optoelectronic device is an organic photovoltaic cell.

12. Use according to Claim 11 **characterized in that** the compound of formula (I) is in the two-component electron selective buffer layer of such derive.

13. A compound of the formula (I)
X-F-(Z-Y-M)ᵣ (I)
**characterized in that**
F is a [60] fullerene,
M represents P(O)(OH)₂,
r represents a number from 2 to 8,
Z represents a group Ar, -CR(R')- or -S-,
n represents a number from 1 to 12,
R and R' independent from each other represent hydrogen or a C₁-C₁₂ carbon chain,
Y represents a C₁-C₁₂ carbon chain,
Ar represents phenyl, biphenyl or naphthyl and
X represents H, Cl or independent from Y a C₁-C₁₂ carbon chain.

14. A compound of the formula (I)
X-F-(Z-Y-M)ᵣ (I)
**characterized in that**
F is a [70] fullerene,
M represents P(O)(OH)₂,
r represents a number from 2 to 8,
Z represents a group Ar,
n represents a number from 1 to 12,
Y represents a C₁-C₁₂ carbon chain,
Ar represents phenyl, biphenyl or naphthyl and
X represents H, Cl or independent from Y a C₁-C₁₂ carbon chain.

15. A two-component electron selection buffer layer **characterized in that** a fullerene compound of formula (I) according to Claim 13 or Claim 14 is used in combination with a metallic or n-type semiconductor metal oxide, preferably ZnO, TiO₂, SnO₂.

16. A two-component electron selection buffer layer according to Claim 15 wherein the metallic or n-type semiconductor metal oxide is indium-tin-oxide, fluorine doped tin-oxide or antimony-tin oxide.
